# EUROPEAN PATENT APPLICATION

(11) **EP 2 027 884 A2**
(43) Date of publication of application: **25.02.2009**
(21) Application number: 08012658.4
(22) Date of filing: 14.07.2008
(51) Int. Cl.: A61M 39/10, A61M 39/12

(54) **Locking connector**

(30) Priority: 24.08.2007 JP 2007217933
(71) Applicant: Covidien AG, 8212 Neuhausen am Rheinfall (CH)
(72) Inventor: Kitani, Ichiro, Tokyo 151-0051 (JP); Funamura, Shigeaki, Tokyo 151-0051 (JP); Satoh, Takashi, Tokyo 151-0051 (JP)
(74) Representative: Olsson, Carl H.S.

(57) **Abstract**

To provide a locking connector with which it is possible to prevent a lock ring from becoming suddenly disconnected from a male luer part, and with which the operation to remove the lock ring from the male luer part is simplified.

[Resolving Means] A locking connector 10 consists of a male luer part 14, a connecting support part 13 which is provided at the rear end of the male luer part 14 and the rear end part of which is connected to a tube 11, and a lock ring 17 which is supported on the connecting support part 13 in a state in which it is prevented from being withdrawn from the tip end of the male luer part 14. Furthermore, guide protrusions 16 extending from the tip end side to the rear end side of the connecting support part 13 while steadily curving in the axial direction of the connecting support part 13 are formed on the outer peripheral surface of the connecting support part 13, and recess parts 17b allowing the entry of the guide protrusions 16 are formed on the inner peripheral surface of the lock ring 17. Then, when the guide protrusions 16 are positioned inside the recess parts 17b, the lock ring 17 can be moved to the tube 11 side from the connecting support part 13.

## Description

The present invention relates to a locking connector which comprises a male luer part which can be fitted to a female luer part provided with a female-side connector, and a lock ring for maintaining the fitted state of the male luer part with the female luer part, by means of engagement with a specific portion of the female-side connector.

### [Prior Art]

Liquids such as drug solutions and blood are conventionally supplied to patients using a liquid transfusion line or a blood transfusion line provided with a tube. A connector for connecting the various tubes which make up the liquid transfusion line or the like is used in such cases. A locking connector provided with a male luer part and a lock ring is one such connector (see Patent Document 1, for example). This locking connection instrument (locking connector) comprises a locknut portion which is fitted to the male luer portion, and a plurality of ribs which are parallel with the direction of liquid flow are provided on the base end side of the male luer portion.

A plurality of groove-shaped parts corresponding to the ribs are also formed on a portion of the locknut portion which is in contact with the base end side of the male luer portion. Consequently, when the locknut portion is being used, the locknut portion is positioned on the outer periphery of the male luer portion so that it can engage with the female-side connector to which the male luer portion is connected. Furthermore, when the locknut is not being used, the locknut portion is moved to the base end side of the male luer portion in a state in which the groove-shaped parts of the locknut portion are fitted into the ribs, whereby it can be retracted to the outer periphery of a tube which is connected to the male luer portion. [Patent Document 1] Japanese Unexamined Patent Application Publication H7-148271

### [Disclosure of the Invention]

However, with the conventional locking connection instrument described above, the locknut portion is unfortunately easily disconnected from the male luer portion not only when the groove-shaped parts of the locknut portion have been intentionally fitted to the ribs of the male luer portion, but also when the positions of the groove-shaped parts of the locknut portion and the ribs of the male luer portion happen to be matching. On the other hand, the locknut portion is difficult to disconnect when a deliberate effort is made to remove the locknut portion from the male luer portion.

The present invention has been devised in view of the situation described above, and it aims to provide a locking connector with which it is possible to prevent a lock ring from becoming suddenly disconnected from a male luer part, and also with which the operation to retract the lock ring to the rear part side of a male side connector so as to disconnect it from the male luer part is simplified.

In order to achieve the aim described above, the structural features of the locking connector according to the present invention lie in the fact that it is a locking connector comprising a male luer part which is able to be fitted to a female luer part provided with a female-side connector which links in communication with a pipe body; a cylindrical connecting support part which is provided at the rear end of the male luer part and of which the rear end part is connected to another pipe body; and a lock ring for maintaining the fitted state of the male luer part with the female luer part by virtue of the fact that it is supported at the connecting support part in a state in which it is prevented from being withdrawn from the tip end side of the male luer part, and it engages with a specific portion of the female-side connector; and in said locking connector, guide protrusions extending from the tip end side at the rear end of the connecting support part while steadily curving in the axial direction of the connecting support part are formed on the outer peripheral surface of the connecting support part, and guided recess parts allowing the entry of the guide protrusions are formed on the inner peripheral surface of the lock ring; and when the guide protrusions are positioned inside the guided recess parts, the lock ring can be moved from the connecting support part to the other pipe body side.

With the locking connector according to the present invention as described above, substantially helical guide protrusions running from the tip end side to the base end side of the connecting support part while steadily curving in the axial direction (in other words, while steadily curving around the axis) are formed on the outer peripheral surface of the connecting support part which is provided at the rear end of the male luer part and of which the rear end part is connected to a pipe body. Guided recess parts allowing the entry of the guide protrusions are then formed on the inner peripheral surface of the lock ring. It should be noted that a "lock ring" in the present invention is a fastening member which is placed on the outer periphery of a male luer part and comprises an annular or cylindrical portion for maintaining a communicating link between the male luer part and a female luer part by engagement with a female-side connector.

Accordingly, if the female-side connector to which the locking connector is connected comprises a part to be engaged with which the lock ring can engage, it is possible to connect the locking connector and the female-side connector in a state in which the male luer part is fitted to the female luer part by insertion of the male luer part into the female luer part to engage the lock ring with the part to be engaged of the female-side connector. The lock ring and the part to be engaged of the female-side connector are preferably engaged by screwing together partner screw fittings.

Furthermore, if the female-side connector to which the locking connector is connected does not comprise a part to be engaged with which the lock ring can engage, the male luer part can be fitted to the female luer part on the female-side connector in a state in which the lock ring is retracted from the connecting support part and is positioned on the outer periphery of a pipe body which is connected to the rear end part of the connecting support part. In this case, the guide protrusions can be easily inserted into the guided recess parts by applying pressure to the rear end of the connecting support part while gently rotating the lock ring around the axis of the connecting support part so as to match the guided recess parts of the lock ring with the guide protrusions of the connecting support part. In this state, the lock ring can be disconnected from the connecting support part and positioned on the outer periphery of the pipe body by moving the lock ring rearwards while further rotating it.

On the other hand, when the lock ring is rotated in the opposite direction to the abovementioned direction with respect to the connecting support part, the tip end part of the substantially helical guide protrusions do not readily engage with the guided recess parts. Furthermore, if pressure is applied to the rear part of the connecting support part along the axial direction without rotating the lock ring, there is virtually no engagement between the tip end part of the guide protrusions and the guided recess parts. If the lock ring is partly rotated in the opposite direction or if pressure is temporarily applied along the axial direction, the lock ring achieves a state in which it is stopped at a certain portion of the guide protrusions, even if the tip end part of the guide protrusions and the guided recess parts engage, because the guide protrusions have a substantially helical shape.

Consequently, it is unlikely that the tip end part of the guide protrusions and the guided recess parts will suddenly engage and that in this state the lock ring will become disconnected from the connecting support part, moving to the outer periphery of the pipe body. In other words, according to the present invention, when a deliberate effort is made to disconnect the lock ring from the connecting support part, the lock ring is easily disconnected from the connecting support part, but at other times the lock ring is not readily disconnected from the connecting support part. Moreover, the guided recess parts can also be configured by obliquely-inclined recess parts conforming to the curvature of the guide protrusions, and by this means the lock ring can be even more reliably prevented from becoming suddenly disconnected from the connecting support part.

Another structural feature of the locking connector according to the present invention lies in the fact that the end part of the guide protrusions at the tip end side of the connecting support part comprises an end surface which is substantially orthogonal to the central axis of the connecting support part.

This means that it is even more unlikely that the tip end part of the guide protrusions and the guided recess parts will suddenly engage and that the lock ring will become disconnected from the connecting support part. In other words, if the tip end surface of the guide protrusions running at an oblique inclination in a substantially helical shape is formed as a surface which is substantially orthogonal to the central axis of the connecting support part, the surface area of that end surface when seen from the front (the axial direction of the connecting support part) is greater than the surface area when seen from the direction in which the guide protrusions run. Consequently, when the lock ring is rotated in a certain direction about the axis with respect to the connecting support part so as to match the guided recess parts of the lock ring with the guide protrusions of the connecting support part, the guide protrusions can easily enter the guided recess parts, but at other times the guide protrusions cannot enter the guided recess parts.

Another structural feature of the locking connector according to the present invention lies in the fact that the guide protrusions are respectively formed on two sides of the outer peripheral surface of the connecting support part, with the central axis of the connecting support part in between, and also the guided recess parts are respectively formed on two sides of the inner peripheral surface of the lock ring, with the central axis of the lock ring in between. This means that the lock ring achieves improved balance with the connecting support part when the lock ring is detached from the connecting support part.

### [Optimum Mode of Embodiment of the Invention]

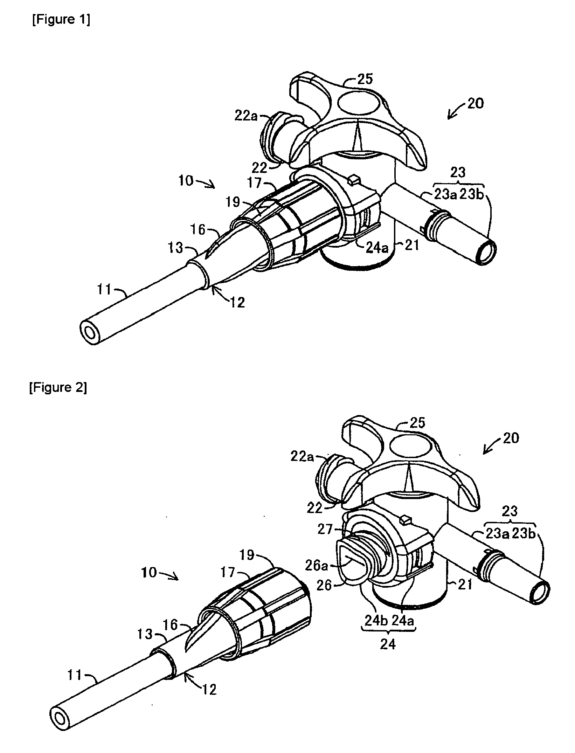

A detailed description of a locking connector according to one mode of embodiment of the present invention will be given below with reference to the figures. Figure 1 shows a state in which a locking connector 10 according to this mode of embodiment is attached to a three-way stopcock 20 which acts as a female-side connector according to the present invention, and Figure 2 shows a state in which the locking connector 10 has been disconnected from the three-way stopcock 20. This locking connector 10 and this three-way stopcock 20 are incorporated in a liquid transfusion line for supplying a drug solution or the like to a patient's body, and the locking connector 10 conveys a drug solution or the like sent from a container or the like (not depicted) housing the drug solution or the like, via a tube 11, to the three-way stopcock 20.

The three-way stopcock 20 allows the drug solution or the like sent from the locking connector 10 to flow to a tube (not depicted) which is connected to the downstream side, and also inhibits this flow; it also allows other drug solutions or the like sent from a container such as a drip tube housing other drug solutions or the like, via a tube (not depicted), to flow to the tube which is connected to the downstream side, and also inhibits this flow. In other words, the three-way stopcock 20 supplies two different types of drug solutions or the like to the patient's body, while the operator switches between the respective flow channels as required.

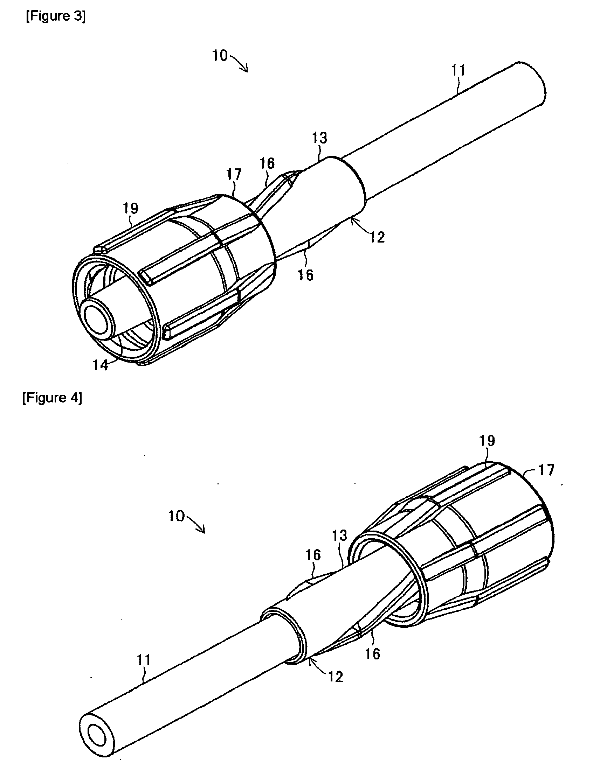

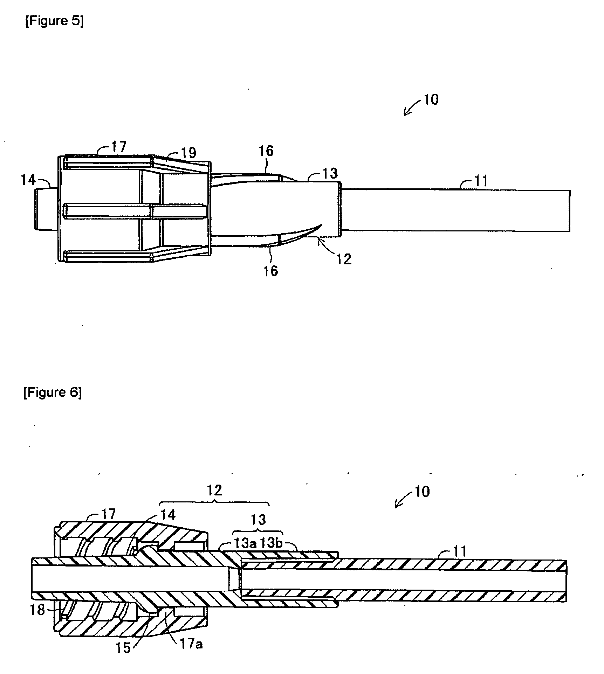

As shown in Figures 3 to 6, the locking connector 10 is configured by a male-side connector main body 12 which is formed as stepped cylinder, and a lock ring 17 which is attached to the outer periphery of the male-side connector main body 12 and is able to rotate about the axis with respect to the male-side connector main body 12, and also which is axially mobile. The male-side connector main body 12 is configured by a support part 13a which is centrally positioned in the axial direction of the male-side connector main body 12 and has a thick cylindrical shape, a cylindrical male luer part 14 which extends away from the tip end part of the support part 13a and is formed to be thinner than the support part 13a, and a cylindrical connecting part 13b which extends rearwards from the rear end part of the support part 13a and is formed to be thinner than the support part 13a.

The outer peripheral surface of the male luer part 14 is formed as a curved surface which tapers gently so that the diameter of the base end side is large, with the diameter becoming smaller towards the tip end side. Furthermore, the inner diameter of the support part 13a is set to be substantially the same as the inner diameter of the male luer part 14, and the outer diameter of the support part 13a is set to be greater than the outer diameter of the male luer part 14. A latch part 15 is then formed around the circumference on the outer periphery at the boundary between the support part 13a and the male luer part 14, in order to prevent the lock ring 17 from moving away to the tip end side of the male luer part 14. The outer diameter of the connecting part 13b is the same as that of the support part 13a, and its inner diameter is greater than that of the support part 13a to the extent of their difference in thickness.

The connecting support part 13 according to the present invention is configured by said support part 13a and connecting part 13b, and a pair of guide protrusions 16 is formed on the outer peripheral surface of the connecting support part 13, running from the tip end side of the support part 13a to the rear end side of the connecting part 13b. The pair of guide protrusions 16 have the same shape, and are formed on both sides of the peripheral surface of the connecting support part 13 maintaining a 180° spacing. Consequently, the relationship between the pair of guide protrusions 16 is such that if one of the guide protrusions 16 is rotated about the axis along the outer peripheral surface of the connecting support part 13, this one of the guide protrusions 16 lies over the other guide protrusion 16. Furthermore, the guide protrusions 16 extend in a helical shape while steadily curving in the circumferential direction with respect to the axial direction of the connecting support part 13.

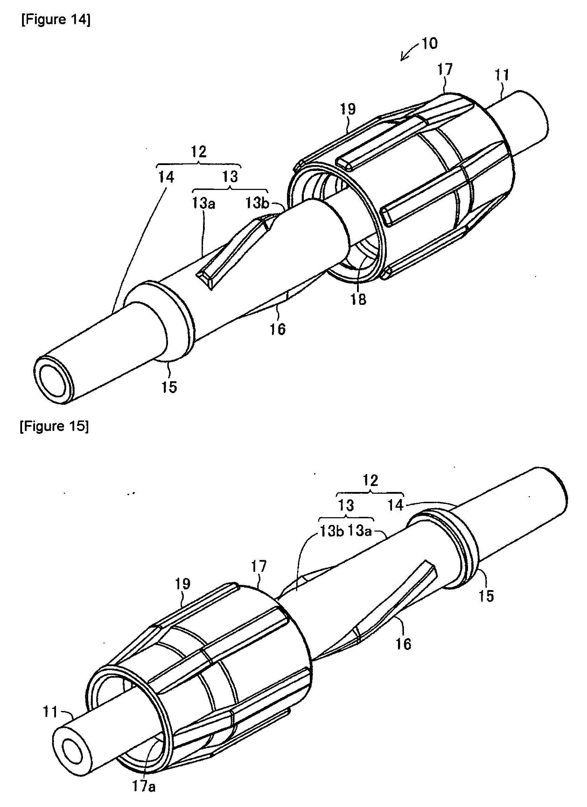

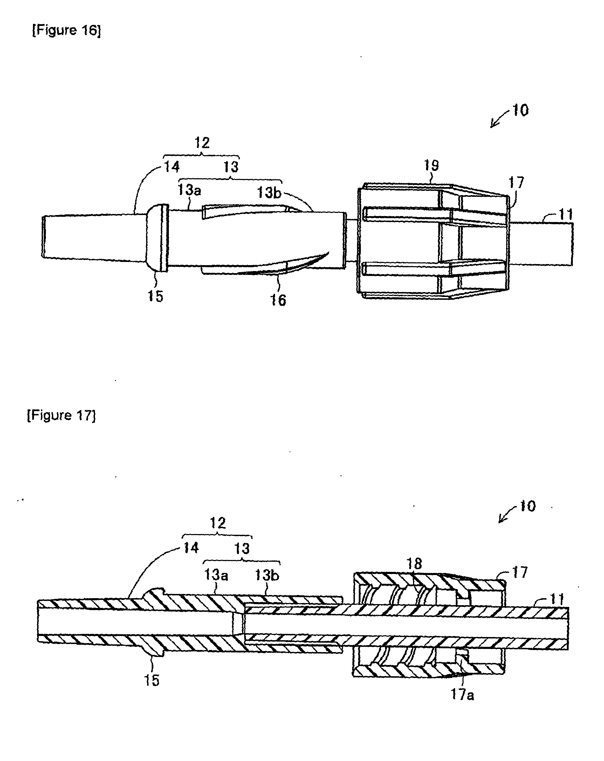

The end surface at the tip end side of the guide protrusions 16 is then formed as a surface which is orthogonal with respect to the central axis of the connecting support part 13 (see Figures 14 and 16), and the rear end part of the guide protrusions 16 is formed as a tapering shape which decreases steadily widthwise and heightwise from the outer peripheral surface of the connecting support part 13. Furthermore, a specified spacing is provided between the end surface at the tip end side of the guide protrusions 16 and the latch part 15 in order to allow movement of the lock ring 17 which will be described hereinafter. Moreover, the angle about the axis of the connecting support part 13 between the tip end part and the rear end part of the guide protrusions 16 can be any angle, but this value is preferably set between 45° and 90°. The male-side connector main body 12 is then joined to the tube 11 which acts as the other pipe body of the present invention by fixedly attaching the tip end part of the tube 11 to the inner peripheral surface of the connecting part 13b.

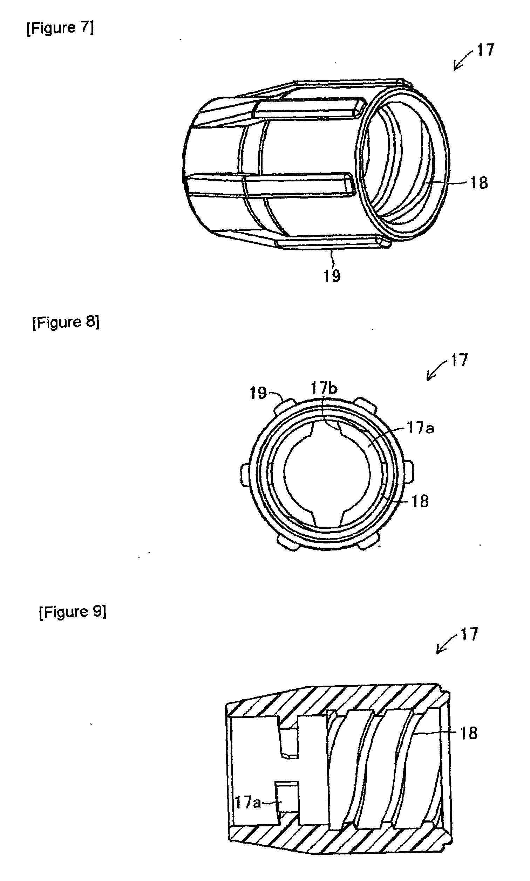

As shown in Figures 7 to 9, the lock ring 17 is formed as a substantially cylindrical shape which is formed as a tapering curved surface in which the circumferential surface of the base end part side steadily tapers towards the rear. A pair of semicircular sliding engagement parts 17a which can slide on the outer peripheral surface of the support part 13a and can also engage with the latch part 15 are formed facing each other along the circumference at a portion on the base end side of the inner peripheral surface of the lock ring 17. The area between the end parts of this pair of sliding engagement parts 17a configures the respective recess parts 17b which act as the guided recess parts according to the present invention, and the guide protrusions 16 of the male-side connector main body 12 are able to enter these. Furthermore, both surfaces of the recess parts 17b are formed as an oblique surface having the same angle of inclination as the respective guide protrusions 16.

Consequently, the lock ring 17 is able to rotate about the axis with respect to the male-side connector main body 12, and is also able to move in the axial direction of the male-side connector main body 12 in a range so that the sliding engagement part 17a abuts the latch part 15 and the guide protrusions 16. Then, when the guide protrusions 16 enter the recess parts 17b, the lock ring 17 can be moved to the rear part side of the connecting support part 13 while it is gently rotated about the axis of the connecting support part 13. Furthermore, a female screw 18 comprising protrusions is formed between a portion whereby a certain distance is maintained from the tip end part of the inner peripheral surface of the lock ring 17 and a central portion in the axial direction. End-of-slide protrusions 19 with a fixed spacing between them in the circumferential direction and running in the axial direction are then formed on the outer peripheral surface of the lock ring 17.

The three-way stopcock 20 is configured by a cylindrical chamber part 21, an upstream pipe 22, a downstream pipe 23 and a merging pipe 24, these being respectively linked to the outer peripheral surface of the chamber part 21, and a flow channel switching part which is disposed between the inside and the outside of the chamber part 21 (only the operating part 25 which includes the flow channel switching part is depicted in the figures). In the state shown in Figures 1 and 2, the chamber part 21 comprises a cylindrical body which has a bottom, disposed so that the axial direction is vertically oriented, and where the lower end part is closed. The upstream pipe 22 is then joined to one of the outer peripheral surfaces of the chamber part 21, and a hole configuring a flow channel and a fitting hole which link in communication with the inside of the chamber part 21 is formed inside said upstream pipe 22.

A portion inside the upstream pipe 22 on the chamber part 21 side configures the flow channel for allowing the passage of liquid, and a portion inside the upstream pipe 22 on the opening side configures the tapered fitting hole which becomes steadily larger in diameter nearer the opening. In other words, a male luer part which is provided at the end part of the tube is fitted to the opening-side portion of the upstream pipe 22, and drug solution or the like which is sent from the tube via the male luer part passes through the flow channel inside the upstream pipe 22 and flows into the chamber part 21. Furthermore, a screw part 22a is formed on the outer peripheral surface of the opening of the upstream pipe 22. The role of this screw part 22a is to allow the lock ring to be screwed on when the male luer part is the same locking connector as the locking connector 10, and the male luer part can be held in a state in which it is fitted to the upstream pipe 22 by screwing the lock ring onto this screw part 22a.

Furthermore, the downstream pipe 23 is joined to the other outer peripheral surface of the chamber part 21, and a flow channel which links in communication with the inside of the chamber part 21 is formed inside said downstream pipe 23. The downstream pipe 23 comprises a base end part 23a which lies on the side of the chamber part 21, and a male luer part 23b which lies on the tip end side of the base end part 23a, and which is narrower than the base end part 23a. Furthermore, the male luer part 23b is formed with a tapering shape in which the tip end portion is narrower than the base end part 23a portion. The male luer part 23b is fitted to a female luer part which is provided at the upstream end of the tube which acts as one of the pipe bodies according to the present invention, and drug solution or the like which is sent from the chamber part 21 is conveyed to the downstream side of the tube via the female luer part.

The merging pipe 24 is joined between the portion of the outer peripheral surface of the chamber part 21 where the upstream pipe 22 is joined, and the portion where the downstream pipe 23 is joined, maintaining an angle of 90° with respect to both the upstream pipe 22 and the downstream pipe 23. The merging pipe 24 comprises a large diameter base end part 24a positioned on the chamber part 21 side which is short in the axial direction and of large diameter, and a connecting part 24b which is provided at the tip end of the large diameter base end part 24a and is of smaller diameter than the large diameter base end part 24a. The connecting part 24b is the portion which configures the female luer part according to the present invention, and its size is set so that it can be inserted into the lock ring 17 in a state in which the male luer part 14 is positioned therein.

It should be noted that here, for ease of description, the right-hand side of the merging pipe 24 in Figures 1 and 2 is taken as the rear or the base end side, while the left-hand side is taken as the front or the tip end side, and the right-hand side of the locking connector 10 in Figures 1 and 2 is taken as the front or the tip end side, while the left-hand side is taken as the rear or base-end side. Furthermore, a rubber stopper 26 is fitted into the connecting part 24b. This rubber stopper 26b consists of an elastic member made of natural rubber, synthetic rubber or elastomer etc. The rubber stopper 26 is then provided with a slit 26a which passes between the inside of the merging pipe 24 and the outside of the merging pipe 24 so as to form part of a flow channel in the merging pipe 24.

Said slit 26a assumes a state in which it is closed due to the elasticity of the rubber stopper 26 when the flow channel in the merging pipe 24 is not being used. Furthermore, when the flow channel in the merging pipe 24 is being used, the male luer part 14 of the locking connector 10 is inserted into the slit 26a of the rubber stopper 26, which enables the locking connector 10 to link in communication with the inside of the chamber part 21. At this time, a sealed state is achieved between the male luer part 14 and the circumferential surface of the slit 26a due to the elasticity of the rubber stopper 26. Furthermore, a male screw 27 consisting of a protrusion is formed on the outer peripheral surface of the connecting part 24b between the tip end side of the connecting part 24b and the large diameter base end part 24a.

The flow channel switching part comprises a valve body (not depicted) which lies inside the chamber part 21, and an operating part 25 which is joined to the upper end of the valve body and is provided with three parts which extend in three directions at intervals of 90°. The valve body rotates about the axis of the chamber part 21 by rotation of the operating part 25. Furthermore, two groove parts of different shape are formed on the outer peripheral surface of the valve body, and by rotating the valve body it is possible to form flow channels between the two groove parts and the inner surface of the chamber part 21 according to this angle, which flow channels link in communication or block the upstream pipe 22, downstream pipe 23 and merging pipe 24, respectively.

As shown in Figure 1, for example, when the respective directions in which each of the parts of the operating part 25 extend match the upstream pipe 22, downstream pipe 23 and merging pipe 24, the upstream pipe 22, downstream pipe 23 and merging pipe 24 are all linked in communication. Furthermore, from the state in Figure 1, when the operating part 25 is rotated through 90° anti-clockwise, and the directions in which two of the parts of the operating part 25 extend match the downstream pipe 23 and the merging pipe 24, respectively, with the remaining part positioned outside of the three-way stopcock 20, the downstream pipe 23 and the merging pipe 24 are linked in communication.

Then, from this state, when the operating part 25 is rotated through a further 90° anti-clockwise, and the directions in which two of the parts of the operating part 25 extend match the upstream pipe 22 and the downstream pipe 23, respectively, with the remaining part positioned outside of the three-way stopcock 20, the upstream pipe 22 and the downstream pipe 23 are linked in communication. In this way it is possible to link in communication or block the upstream pipe 22, downstream pipe 23 and merging pipe 24, respectively, by rotating the operating part 25 to rotate the valve body. Moreover, a reverse-flow prevention wall or the like is provided inside the chamber part 21, and this prevents drug solution or the like from flowing to the upstream pipe 22 or merging pipe 24 side from the downstream pipe 23 side, even if the upstream pipe 22 or the merging pipe 24 is linked in communication with the downstream pipe 23.

When a liquid transfusion line set provided with the locking connector 10 and the three-way stopcock 20 configured in the manner described above is used, the male-side connector which is provided at the downstream end of a tube extending from a container which houses drug solution or the like is firstly connected to the upstream pipe 22 of the three-way stopcock 20.
Furthermore, the female-side connector which is provided at the upstream end of a tube which has a piercing member such as an indwelling needle for piercing the patient's body so as to remain indwelling connected to its downstream end is connected to the downstream pipe 23. Next, the tip end of the locking connector 10 is moved close and opposite to the tip end of the merging pipe 24 of the three-way stopcock 20, and the tip end of the male luer part 14 of the locking connector 10 is pushed into the slit 26a of the rubber stopper 26.

Next, the tip end of the connecting part 24b of the merging pipe 24 is positioned inside the lock ring 17, and the male screw 27 and the female screw 18 come into contact, after which the lock ring 17 is rotated about a specific axis, whereby the male screw 27 and the female screw 18 screw together. By means of this, the merging pipe 24 and the locking connector 10 link in communication in a state of linkage which is adequate for the purpose of preventing leakage of liquid. Furthermore, a container housing another drug solution or the like is connected to the upstream end of the tube 11 which is connected to the locking connector 10. The piercing member at the downstream end of the tube which is connected to the downstream pipe 23 then pierces the patient's body and remains indwelling. Next, the container at the upstream end of the tube which is connected to the upstream pipe 22 and the container at the upstream end of the tube 11 are opened, and a state is achieved whereby drug solutions or the like can be supplied from both containers. Then, by operating the operating part 25, specified drug solutions or the like are supplied to the patient's body in the required amounts.

Furthermore, when the lock ring 17 is not used, the male luer part 14 of the locking connector 10 is directly fitted to the inner peripheral surface of the female luer part. The lock ring 17 is then retracted to the outer periphery of the tube 11. In this case, from the state shown in Figures 3 to 6, the lock ring 17 is rotated in one direction (anti-clockwise in the state shown in Figure 3) with respect to the male-side connector main body 12, while being gently pushed rearwards. Then, when the recess parts 17b of the lock ring 17 reach the position of the tip end part of the guide protrusions 16, the tip end part of the guide protrusions 16 enters the recess parts 17b.

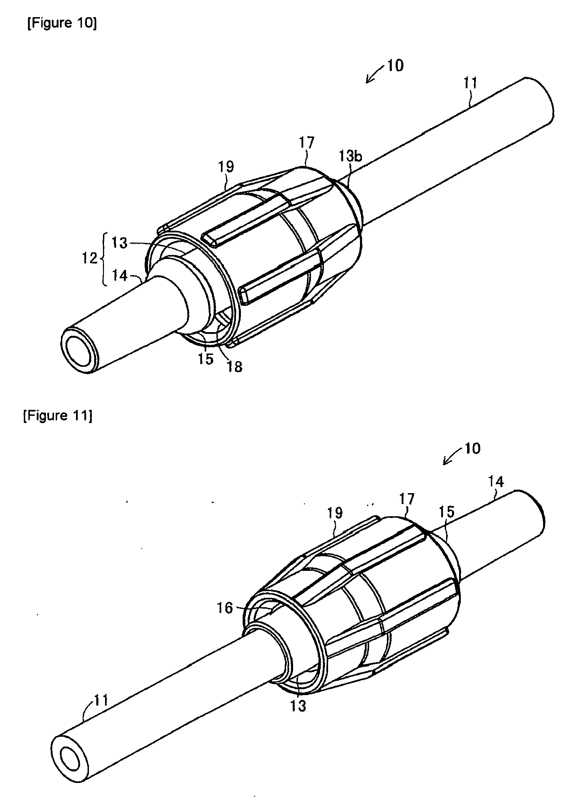

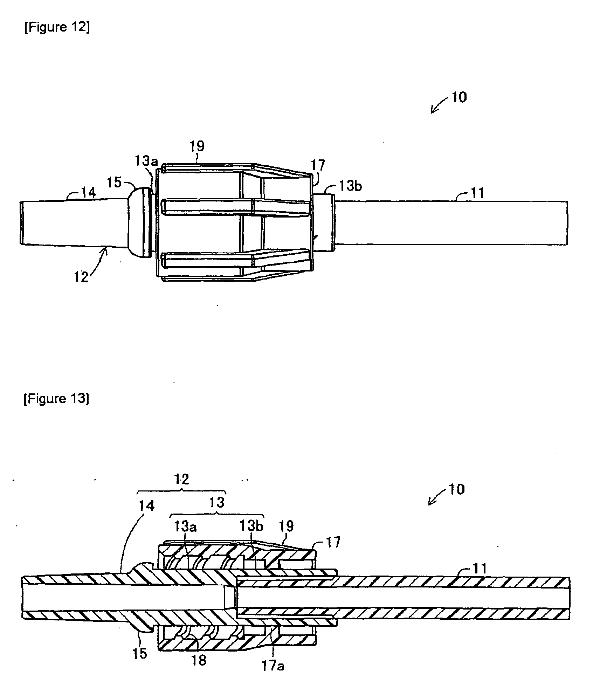

In this state, if the lock ring 17 is further rotated with respect to the male-side connector main body 12, the lock ring 17 is moved to the rear part side of the male-side connector main body 12 along the guide protrusions 16, and the state shown in Figures 10 to 13 is achieved. Then, if the lock ring 17 is further rotated with respect to the male-side connector main body 12, the engagement between the recess parts 17b and the guide protrusions 16 is released, and the lock ring 17 is disconnected from the male-side connector main body 12. By means of this, the lock ring 17 reaches a state in which it is retracted to the outer periphery of the tube 11, as shown in Figures 14 to 17. Moreover, the male screw which can be screwed together with the female screw 18 of the lock ring 17 is provided at the rear end part of the tube 11, which means that the lock ring 17 can be fixed to the rear end part of the tube 11. By means of this, the lock ring 17 does not get in the way during the operation when the male luer part 14 and the female luer part are fitted together and during use of the transfusion line set.

As described above, with the locking connector 10 according to this mode of embodiment, helical guide protrusions 16 extending from the tip end side to the base end side of the connecting support part 13 while steadily curving in the axial direction are formed on the outer peripheral surface of the connecting support part 13, and recess parts 17b allowing the entry of the guide protrusions 16 are formed on the inner peripheral surface of the lock ring 17. Accordingly, if the female-side connector to which the locking connector 10 is connected does not comprise a male screw with which the lock ring 17 can be screwed, the male luer part 14 can be fitted to the female luer part on the female-side connector in a state in which the lock ring 17 is retracted from the male-side connector main body 12 and is positioned on the outer periphery of the tube 11 which is connected to the rear end part of the connecting support part 13.

In this case, the guide protrusions 16 can be easily inserted into the recess parts 17b by applying pressure to the rear end of the connecting support part 13 while gently rotating the lock ring 17 around the axis of the connecting support part 13 so as to match the recess parts 17b of the lock ring 17 with the guide protrusions 16 of the connecting support part 13. In this state, the lock ring 17 can be disconnected from the male-side connector main body 12 and positioned on the outer periphery of the tube 11 by moving the lock ring 17 rearwards while further rotating it.

In this case, when the lock ring 17 is rotated in the opposite direction to the abovementioned direction, or when pressure is applied to the rear part of the connecting support part 13 without rotating the lock ring, there is virtually no engagement between the tip end part of the guide protrusions 16 and the recess parts 17b. Unless a rearwards urging force and a rotational force are applied to the lock ring 17, it achieves a state in which it is stopped at a certain portion of the guide protrusions 16 even if the tip end part of the guide protrusions 16 and the recess parts 17b temporarily or accidentally engage, because the guide protrusions 16 have a substantially helical shape. Consequently, the guide protrusions 16 and the recess parts 17b do not suddenly engage and in this state the lock ring 17 does not become disconnected from the male-side connector main body 12, moving to the outer periphery of the tube 11.

Furthermore, in this mode of embodiment, the tip end surface of the guide protrusions 16 is formed as a surface which is substantially orthogonal to the central axis of the connecting support part 13, and therefore the surface area of that end surface when seen from the central axis of the connecting support part 13 is greater than the surface area when seen from the direction in which the guide protrusions 16 run. Consequently, when the lock ring 17 is rotated in a certain direction about the axis so as to match the recess parts 17b of the lock ring 17 with the guide protrusions 16, the guide protrusions 16 can easily engage with the recess parts 17b, but at other times the guide protrusions 16 cannot engage with the recess parts 17b. In this way, according to this mode of embodiment, when a deliberate effort is made to disconnect the lock ring 17 from the male-side connector main body 12, the lock ring 17 is easily disconnected, but at other times the lock ring 17 is not readily disconnected from the male-side connector main body 12.

In addition, provision is made for a pair of guide protrusions 16, and therefore an effect is obtained whereby the lock ring 17 achieves improved balance with the connecting support part 13 when the lock ring 17 is moved along the guide protrusions 16. Furthermore, if the female-side connector to which the locking connector 10 is connected is the three-way stopcock 20 described above, the locking connector 10 can be connected to the merging pipe 24 of the three-way stopcock 20 in a state in which the male luer part 14 is fitted into the rubber stopper 26 of the merging pipe 24, by pushing the male luer part 14 into the slit 26a of the rubber stopper 26 which is provided in the merging pipe 24 of the three-way stopcock 20 and screwing the female screw 18 of the lock ring 17 onto the male screw 27 of the merging pipe 24.

Furthermore, the locking connector according to the present invention is not limited to the mode of embodiment described above, and appropriate modifications can be implemented. For example, in the mode of embodiment described above, the female-side connector consists of the three-way stopcock 20, and the locking connector 10 is connected to the merging pipe 24 of the three-way stopcock 20, but the upstream pipe 22 or a pipe member similar to the upstream pipe 22 may be used as the female connector. In this case, a male screw which is similar to the male screw 27 is provided on the outer periphery on the opening side of the pipe member such as the upstream pipe 22, instead of the linking screw part 22a. Further appropriate modifications within the technical scope of the present invention may also be made to other components of the locking connector according to the present invention.

### [Brief Description of the Figures]

[Figure 1] is an oblique view showing a state in which the locking connector according to a mode of embodiment of the present invention is connected to the three-way stopcock;
[Figure 2] is an oblique view showing a state in which the locking connector has been disconnected from the three-way stopcock;
[Figure 3] is an oblique view showing the state of the locking connector seen obliquely from the front;
[Figure 4] is an oblique view showing the state of the locking connector seen obliquely from the rear;
[Figure 5] is a front view of the locking connector;
[Figure 6] is a cross-sectional view of the locking connector;
[Figure 7] is an oblique view showing the lock ring;
[Figure 8] is a side view of the lock ring;
[Figure 9] is a cross-sectional view of the lock-ring;
[Figure 10] is an oblique view showing the state of the locking connector where the lock ring is to be disconnected, seen obliquely from the front;
[Figure 11] is an oblique view showing the state of the locking connector where the lock ring is to be disconnected, seen obliquely from the rear;
[Figure 12] is a front view of the locking connector where the lock ring is to be disconnected;
[Figure 13] is a cross-sectional view of the locking connector where the lock ring is to be disconnected;
[Figure 14] is an oblique view of the locking connector where the lock ring has been removed, seen obliquely from the front;
[Figure 15] is an oblique view of the locking connector where the lock ring has been removed, seen obliquely from the rear;
[Figure 16] is a front view of the locking connector where the lock ring has been removed; and
[Figure 17] is a cross-sectional view of the locking connector where the lock ring has been removed.

### [Explanation of Symbols]

10...locking connector, 11...tube, 13...connecting support part, 14...male luer part, 15...latch part, 16...guide protrusion, 17...lock ring, 17b...recess part, 20...three-way stopcock, 24...merging pipe.

## Claims

1. Locking connector comprising a male luer part which is able to be fitted to a female luer part provided with a female-side connector which links in communication with a pipe body; a cylindrical connecting support part which is provided at the rear end of the abovementioned male luer part and of which the rear end part is connected to another pipe body; and a lock ring for maintaining the fitted state of the abovementioned male luer part with the abovementioned female luer part by virtue of the fact that it is supported at the abovementioned connecting support part in a state in which it is prevented from being withdrawn from the tip end side of the abovementioned male luer part, and it engages with a specific portion of the abovementioned female-side connector,
said locking connector being **characterized in that** guide protrusions extending from the tip end side at the rear end of the abovementioned connecting support part while steadily curving in the axial direction of the abovementioned connecting support part are formed on the outer peripheral surface of the abovementioned connecting support part, and guided recess parts allowing the entry of the abovementioned guide protrusions are formed on the inner peripheral surface of the abovementioned lock ring; and when the abovementioned guide protrusions are positioned inside the abovementioned guided recess parts, the abovementioned lock ring can be moved from the abovementioned connecting support part to the abovementioned other pipe body side.

2. Locking connector according to Claim 1, in which the end part of the abovementioned guide protrusions at the tip end side of the abovementioned connecting support part comprises an end surface which is substantially orthogonal to the central axis of the abovementioned connecting support part.

3. Locking connector according to Claim 1 or 2, in which the abovementioned guide protrusions are respectively formed on two sides of the outer peripheral surface of the abovementioned connecting support part, with the central axis of the abovementioned connecting support part in between, and also the abovementioned guided recess parts are respectively formed on two sides of the inner peripheral surface of the abovementioned lock ring, with the central axis of the abovementioned lock ring in between.
